(19) Europäisches Patentamt
European Patent Office
Office européen des brevets

(11) Numéro de publication: **0 303 530 B1**

# (12) FASCICULE DE BREVET EUROPEEN

(45) Date de publication du fascicule du brevet: **18.10.95**

(51) Int. Cl.[6]: **C08G 69/20**, C08J 3/14, A61K 7/035

(21) Numéro de dépôt: **88402002.5**

(22) Date de dépôt: **01.08.88**

(54) **Poudre de polyamide constituée de particules à structure "rose des sables" - procédé d'obtention de la poudre de polyamide.**

(30) Priorité: **11.08.87 FR 8711422**

(43) Date de publication de la demande:
**15.02.89 Bulletin 89/07**

(45) Mention de la délivrance du brevet:
**18.10.95 Bulletin 95/42**

(84) Etats contractants désignés:
**AT BE CH DE FR GB IT LI LU NL SE**

(56) Documents cités:
**FR-A- 2 576 602**

**PARFUMS, COSMETIOUES, AROMES, no. 52, août/septembre 1983, pages 83-85; R. KERAUDY: "Les poudres polyamides dans les formulations cosmétiques"**

(73) Titulaire: **ELF ATOCHEM S.A.**
**4 & 8, Cours Michelet**
**La Défense 10**
**F-92800 Puteaux (FR)**

(72) Inventeur: **Hilaire, Jean-Claude**
**5, Avenue Gratiane**
**F-64000 Pau (FR)**
Inventeur: **Guerin, Roland**
**232, Avenue Jean Mermoz**
**F-64000 Pau (FR)**

(74) Mandataire: **Foiret, Claude et al**
**ELF ATOCHEM S.A.**
**Département Propriété Industrielle**
**La Défense 10 - Cedex 42**
**F-92091 Paris-La-Défense (FR)**

**EP 0 303 530 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne une poudre de polyamide constituée de particules élémentaires poreuses à structure "rose des sables". La particularité de ces particules est de posséder un haut degré d'absorption du fait du nombre important de leurs pores et de leurs volumes. Ces propriétés des particules ont pour conséquence de conduire à une poudre de polyamide de grande surface spécifique et de faible densité apparente.

La poudre de polyamide est obtenue par polymérisation anionique de lactame dans un milieu solvant en présence d'au moins une alkylène amide. La particularité de structure des particules est obtenue par initiation de la polymérisation dans un milieu solvant sursaturé en lactame à la température d'initiation.

Dans le FR 2 576 602 est décrit un procédé de fabrication de poudre de polyamide par polymérisation anionique de lactame en solution en présence d'une alkylène bisamide. Selon la technique de ce brevet qui consiste, en présence d'une alkylène bisamide, à dissoudre la totalité du lactame dans le solvant préalablement à l'initiation de la polymérisation, on obtient des poudres de granulométrie et de poids moléculaire contrôlés. Les particules obtenues possèdent une faible surface spécifique : inférieure à 9 $m^2/g$, avec pour conséquence une très faible porosité.

Dans la revue "Parfums, Cosmetiques, Arômes" N° 53, Août/Septembre 1983, page 84, ou mentionne dans le tableau concernant les caracteristiques de poudre polyamide que la surface specifique est comprise entre 0,5 et 9 $m^2/g$.

Les particules élémentaires poreuses de la poudre de polyamide de la présente invention ont une surface spécifique supérieure à 9 m2/g et un diamètre moyen compris entre 1 et 20 $\mu m$ et sont de forme sensiblement sphéroïdale à structure lamellaire dont les lamelles d'épaisseur inférieure à 0,2 $\mu m$, liées les unes aux autres constituent des cavités de formes géometriques évoluant entre la forme conique et la forme pyramidale, les sommets de ces formes géométriques étant dirigés vers le centre des particules et le volume poreux interne aux particules élémentaires étant supérieur à 0,3 m3/g dans le domaine des rayons médians des pores compris entre 0,02 et 0,4 $\mu m$.

Ces particules élémentaires sphéroïdales ont un diamètre moyen compris entre 1 et 20 microns et habituellement compris entre 2 et 10 microns. Elles sont caractérisées en ce qu'elles possèdent un grand volume poreux. Le volume poreux interne aux particules est généralement supérieur à 0,3 $cm^3/g$, et le plus souvent supérieur à 1 $cm^3/g$, dans le domaine de rayons médians des pores compris entre 0,02 et 0,4 $\mu m$. Le rayon médian des pores de ces particules, les cavités définies précédemment étant assimilées à des cylindres, est habituellement compris entre 0,09 et 0,16 $\mu m$. Ce volume poreux est déterminé par intrusion de mercure sous pression variable selon la loi de WASHBURN

$$R = \frac{2\ \gamma\ \mathrm{Cos}\ \theta}{P}$$

R = le rayon du pore cylindrique
$\gamma$ = tension interfaciale Hg/solide
$\theta$ = angle de contact Hg/solide
P = pression d'intrusion du Hg
(Proc. Nat. Acad. Sci. USA 7, 115 - 1921).

Du fait du nombre et du volume des pores des particules élémentaires constituant la poudre, il résulte que cette dernière possède une surface spécifique supérieure à 9 $m^2/g$ et très couramment comprise entre plus de 9 et 30 $m^2/g$. Cette surface spécifique est déterminée selon la méthode BET classique.

Toujours du fait de leur structure les particules élémentaires possèdent une très forte capacité d'absorption. Cette nouvelle propriété est caractérisée en ce que la poudre constituée de ces particules élémentaires absorbe au minimum 90 % et généralement plus de 120 % de son poids d'huile de lin. Les valeurs d'absorption sont déterminées selon la norme ASTM D 281-31 relative à l'essai de la prise d'huile des pigments. Cette caractéristique d'absorption est particulièrement intéressante dans le domaine des cosmétiques, des peintures, de la pharmacie, de la microencapsulation et autres où l'on cherche à faire absorber aux poudres le maximum d'adjuvants ; c'est par exemple le cas en cosmétologie pour les poudres de beauté ou dans l'industrie des peintures où l'on souhaite incorporer le maximum d'éléments actifs dans le minimum de support.

A cette très forte capacité d'absorption s'associe la lenteur de désorption. La constatation de ce dernier phénomène renforce l'intérêt des poudres contenant ces particules spécifiques dans les applications citées.

La poudre de polyamide formée initialement de ces particules contient généralement au minimum 90 % et le plus souvent 95 % en poids de particules possédant les caractéristiques définies précédemment. Une autre des caractéristiques de la poudre est de posséder une distribution granulométrique restreinte. Cette granulométrie peut être déterminée au compteur COULTER suivant la norme NF x 11-670 et 671. A partir de la courbe des fréquences cumulées en diamètre décroissant, il est possible de calculer les diamètres d50 et les diamètres D84.13 et d15.87 correspondant à une déviation standard de part et d'autre de la médiane

d50. L'étalement granulométrique ou la dispersion granulométrique est défini par le rapport

$$\sigma^2 = \frac{d16.}{d84}$$

La dispersion granulométrie de la poudre est habituellement comprise entre 1,2 et 2,5.

Du fait de l'importante porosité des particules, la poudre de polyamide possède une faible densité apparente. Habituellement selon la norme ISO R787/11 la densité apparente de la poudre non tassée est comprise entre 0,12 et 0,22, la densité apparente tassée étant comprise entre 0,22 et 0,30.

Le procédé d'obtention des poudres de polyamide est, de façon générale, classique. Il est basé sur la polymérisation anionique des lactames fondée principalement sur l'utilisation comme catalyseur d'un métal alcalin ou d'un de ses composés tel que le sodium ou un de ses composés comme l'hydrure de sodium ou le méthylate de sodium. Est également utilisé dans ce type de polymérisation un activateur choisi par exemple parmi les lactames-N-carboxyanilides, les isocyanates, les carbodiimides, les cyanimides, les acyllactames, les triazines, les urées, les imides-N-substituées.

Les lactames pris comme monomères sont de préférence, industriellement dans l'état actuel de la technique, le lauryl lactame, le caprolactame, l'oenantholactame, et le capryllactame ou leurs mélanges.

Egalement de façon connue, la polymérisation des lactames s'effectue en milieu solvant, inerte vis à vis des composants de la réaction et dans le mécanisme de réaction, en présence d'au moins une amide dont une est toujours une N,N'-alkylène bisamide.

Parmi les N,N'-alkylène bisamides particulièrement recommandées sont citées les N,N'-alkylène bisamides d'acides gras et mieux encore :

. la N,N'-éthylène bistéréamide de formule

$$\begin{array}{l} CH_2 - NH - CO - C_{17}H_{35} \\ | \\ CH_2 - NH - CO - C_{17}H_{35} \end{array}$$

. la N,N'-éthylène bisoléamide de formule

$$\begin{array}{l} CH_2 - NH - CO - C_{17}H_{33} \\ | \\ CH_2 - NH - CO - C_{17}H_{33} \end{array}$$

. les N,N'-éthylène bispalmitamide, gadoléamide, cetoléamide et érucamide, la N,N'-dioleyladipamide et la N,N'-diérucylamide.

La quantité de N,N'-alkylène bisamide mise en oeuvre est de l'ordre de 0,001 à 4 moles, et mieux de 0,075 à 2 moles pour 100 moles de lactame.

Le but de l'ajout de N,N'-alkylène bisamide dans le milieu réactionnel est de provoquer un ralentissement de la réaction pour produire une poudre de granulométrie très resserrée sans encrassement du réacteur.

Tous les solvants des lactames conviennent au milieu réactionnel dans la mesure où ils sont inertes vis à vis des réactifs et ne participent pas à la réaction de polymérisation. Le solvant le plus utilisé essentiellement pour des raisons économiques est une coupe d'hydrocarbure paraffinique, mélange d'isoparaffine, de N-paraffine et de cycloparaffine, dont la plage d'ébullition se situe entre 140 et 170 °C. Toutefois pour obtenir les particules et les poudres de l'invention les isoparaffines, de préférence contenant de 6 à 12 atomes de carbone dans la molécule, sont particulièrement recommandées. Le point d'ébullition de ces isoparaffines est en général d'au moins 120 °C.

La poudre de polyamide selon l'invention peut être obtenue par polymérisation anionique en milieu solvant de lactame en présence de catalyseur, d'activateur et d'au moins une amide, dont une est une N, N'-alkylène bisamide, ladite polymérisation étant initiée avec une quantité de lactame et d'amide de telle que le solvant est en état de sursaturation à la température d'initiation.

Différents moyens permettent de sursaturer le solvant du milieu réactionnel en lactame. L'un des moyens peut consister à saturer le solvant en lactame et amide, avant ajout du catalyseur, à une température supérieure à celle d'initiation, puis à l'abaisser pour l'initiation de la polymérisation.

Un autre moyen, objet de l'invention consiste à sensiblement saturer le solvant en lactame et amide à la température d'initiation de la polymérisation et à additionner aux réactifs, avant l'initiation, une amide primaire de façon à diminuer la solubilité du lactame. Cette amide primaire, contenant de préférence de 12 à 22 atomes de carbone dans sa molécule, peut être choisie parmi : l'oléamide, la N-stéramide, l'isostéramide, l'érucamide. La quantité d'amide primaire à mélanger aux autres réactifs est de façon convenable inférieure à 0,5 mole pour 100 moles de lactame.

Les limites de sursaturation du solvant ne sont pas critiques. La limite inférieure peut être telle que, le milieu se trouvant à l'état monophasique, mais métastable,à la température d'initiation, il suffise de l'apport de quelques cristaux d'un des éléments réactifs solubles dans le solvant pour troubler le milieu. La polymérisation peut également être initiée à partir d'un milieu biphasique, une partie du lactame en excès se trouvant à l'état

solide dans le milieu ; dans ces conditions au fur et à mesure de la précipitation des particules de polyamide formées, le lactame en excès se dissout dans le milieu avant polymérisation.

A titre d'exemple la polymérisation peut s'effectuer de façon classique en mettant en contact dans un réacteur le solvant et une quantité de lactame et d'amide telle que le solvant se trouve en état de sursaturation à la température ultérieure d'initiation de la polymérisation.

Toute trace d'humidité étant à proscrire dans la polymérisation, il est recommandé, d'utiliser des réactifs parfaitement anhydre, ou de procéder de façon connue à leur séchage avant l'initiation de la polymérisation.

Le mélange, de préférence sous agitation et sous atmosphère inerte est amené à la température d'initiation, puis sont ajoutés le catalyseur anionique et l'activateur simultanément ou séparément et en une seule fois ou progressivement.

La quantité de catalyseur introduite peut varier de 0,8 à 3 moles pour 100 moles de lactame. Le taux d'activateur introduit peut varier de 2 à 8 moles pour 100 moles de lactame.

La température d'initiation et de polymérisation des lactames est généralement comprise entre 80 et 130°C, la température la plus courante étant voisine de 100°C.

Il est possible d'introduire dans le milieu réactionnel des germes de cristallisation. Ces germes de cristallisation se présentent sous forme de charge finement divisée. Ces charges peuvent être organiques comme de la poudre de polyamide, et mieux des particules selon l'invention préparées antérieurement, ou minérales comme de la silice ou du talc. Il importe que cette charge n'apporte aucune trace d'eau, en particulier quand la silice est utilisée elle doit être soigneusement déshydratée.

Les exemples suivants illustrent l'invention sans la limiter.

Dans ces exemples, les essais ont été réalisés dans un réacteur d'une capacité de 5 litres, muni d'un agitateur à pales, d'une double enveloppe dans laquelle circule de l'huile de chauffage, d'un système de vidange par le fond et d'un sas d'introduction des réactifs balayé à l'azote sec.

Un dispositif de distillation azéotropique sous vide permet d'éliminer toute trace d'eau du milieu réactionnel.

Le solvant utilisé est une coupe d'hydrocarbure paraffinique dont la plage d'ébullition se trouve entre 130 et 160°C.

La granulométrie, diamètre moyen des particules, est mesurée au compteur COULTER.

Pour des raisons de commodité, on désigne

- la N,N'-éthylène bis stéaramide par l'abréviation EBS
- la n-stéaramide par l'abréviation nST
- l'iso-stéaramide par l'abréviation iST

EXEMPLE 1

On introduit dans le réacteur, maintenu sous un léger courant d'azote, 3040 ml de solvant, puis successivement 1087 g de lauryl lactame sec, 37,4 g d'EBS, 0,55 g de nST et 4,3 g de silice finement divisée et déshydratée.

Après avoir mis en route l'agitation à 720 t/minute, on chauffe progressivement jusqu'à 100°C puis on distille, sous un vide de 26660 Pa, 200 ml de solvant afin d'entraîner, par azéotropie, toute trace d'eau éventuellement présente.

Après retour à la pression atmosphérique, on introduit alors rapidement sous azote le catalyseur anionique, 1,95 g d'hydrure de sodium à 80 % de pureté dans de l'huile, et on laisse sous agitation, toujours sous courant d'azote, pendant 60 minutes.

Ensuite, on ramène la température à 100°C et grâce à une petite pompe doseuse, on injecte en continu dans le milieu réactionnel l'activateur choisi : l'isocyanate de stéaryle. La quantité d'isocyanate ainsi injectée est de 33 g en 6 heures puis de 21,8 g pendant 2 heures. Parallèlement, la température est maintenue à 100°C pendant les 6 premières heures ; puis montée et maintenue à 110°C pendant encore 3 heures, soit 1 heure encore après la fin de l'introduction de l'isocyanate.

La polymérisation est alors terminée. On refroidit le réacteur à 90°C et soutire par le fond la bouillie de poudre et de solvant.

Après essorage et séchage, on obtient une poudre de polyamide 12 de granulométrie comprise entre 5,3 et 10,5 microns, le diamètre moyen des particules étant de 8 $\mu$m. La densité apparente non tassée de 0,20 et la densité apparente tassée est de 0,27 et la surface spécifique BET de 9,4 $m^2$/g. Le volume poreux des particules est de 2,04 $cm^3$/g. La poudre absorbe 180 % de son poids d'huile de lin.

Le produit obtenu est illustré en Figure 1 par sa reproduction photographique.

EXEMPLE 2

Le mode opératoire est identique à celui de l'Exemple 1, mais le solvant utilisé n'est plus une coupe paraffinique quelconque mais est constitué uniquement d'isoparaffines C8 à C10, lesquelles ont un pouvoir solvant beaucoup plus faible vis à vis du lauryl lactame. La poudre obtenue dans ces conditions possède une granulométrie comprise entre 4 et 8,6 microns, et une structure "rose des sables". La surface spécifique BET est de 10,2 $m^2$/g, la densité apparente non tassée de 0,18 et la densité apparente tassée de 0,26. Le diamètre

moyen des particules est de 5,8 µm et leur volume poreux est de 2,33 $cm^3/g$. La poudre absorbe 220 % de son poids d'huile de lin.

Le produit obtenu est illustré en Figure 2 par sa reproduction photographique.

EXEMPLE 3

On introduit dans le réacteur 2450 ml de solvant, puis successivement 873 g de lauryl lactame, 0,44 g d'iST, 30 g d'EBS et 17,4 g de silice.

On chauffe sous une agitation de 720 t/minute, jusqu'à 110 °C, puis on distille sous un vide de 26660 Pa, 200 ml de solvant. Après retour à la pression atomosphérique, on introduit sous azote 1,56 g d'hydrure de sodium à 80 % de pureté et on maintient sous azote à 110 °C pendant 30 minutes. On réduit la température à 95 °C puis au moyen d'une petite pompe doseuse, on introduit progressivement de l'isocyanate de stéaryle selon le programme suivant :

- 20 g d'isocyanate pendant 6 heures à 95 °C,
- 30 g d'isocyanate pendant 2 heures 30 à 110 °C.

Une fois cette introduction terminée, la température est maintenue à 110 °C pendant encore 1 heure. La réaction est alors terminée. Après refroidissement à 90 °C, décantation et séchage, la poudre de polyamide 12 obtenue présente les caractéristiques suivantes :

- granulométrie comprise entre 3,4 et 7 µm
- diamètre moyen des particules : 5,3 µm
- surface spécifique : 17 $m^2/g$
- densité apparente non tassée : 0,14
- densité apparente tassée : 0,25
- volume poreux : 2,52 $cm^3/g$
- Absorption de l'huile de lin : 200 % en poids

EXEMPLE 4

On introduit 2440 ml de solvant, 873 g de dodécalactame, 0,4 g d'érucamide, 30 g d'EBS et 17,5 g de silice. Toujours comme dans les essais précédents, on distille azéotropiquement 200 ml de solvant sous 26660 Pa à 110 °C. On refroidit à 100 °C et on ajoute 1,71 g d'hydrure de sodium à 80 % sous azote. Après 1 heure, on règle l'agitation à 720 t/minute et on injecte l'isocyanate de stéaryle selon le programme suivant :

- 20 g d'isocyanate pendant 6 heures à 95 °C,
- 30 g d'isocyanate pendant 2 heures 30 à 110 °C

puis on maintient la température à 110 °C pendant 1 heure.

Après refroidissement et séchage, on obtient une poudre de viscosité 0,75 et de granulométrie comprise entre 3,6 et 8 µm avec un diamètre moyen des particules de 5,4 µm et une surface spécifique BET de 15,6 $m^2/g$.

La densité apparente non tassée est de 0,17, la densité apparente tassée de 0,26. Les particules de la poudre se présentent sous l'aspect "rose des sables" et ont un volume poreux de 2,13 $cm^3/g$. La poudre absorbe 220 % de son poids d'huile de lin.

EXEMPLE 5

On introduit dans le réacteur 3040 ml de solvant, puis, successivement 1304 g de lauryl lactame, 45 g d'EBS et 5,2 g de silice.

On chauffe sous une agitation de 720 t/minute jusqu'à 110 °C puis on distille sous un vide de 26660 Pa 200 ml de solvant. Après retour à la pression atomosphérique, on introduit sous azote 2,34 g d'hydrure de sodium à 80 % et on maintient sous azote à 110 °C pendant 30 minutes. On réduit ensuite la température à 100 °C. On introduit alors progressivement l'isocyanate de stéaryle suivant le programme suivant :

- 30 g d'isocyanate pendant 6 heures à 100 °C
- 36 g d'isocyanate pendant 2 heures à 110 °C

et on maintient la température à 110 °C pendant 1 heure.

Après refroidissement à 90 °C, décantation et séchage, on obtient de la poudre de polyamide 12 présentant les caractéristiques suivantes :

- granulométrie comprise entre 4,8 et 9,3 microns
- diamètre moyen des particules : 6,7 µm
- surface spécifique BET : 9,3 $m^2/g$
- densité apparente non tassée : 0,20
- densité apparente tassée : 0,29
- volume poreux : 2,07 $cm^3/g$
- absorption d'huile de lin : 180 % en poids

Les particules de la poudre se présentent sous l'aspect "rose de sables".

EXEMPLE 6

On introduit dans le réacteur 2240 ml de solvant, puis successivement 1087 g de caprolactame, 18,7 g d'EBS et 21,9 g de silice. On chauffe comme précédemment jusqu'à 110 °C sous une agitation de 720 t/minute, puis on distille sous un vide 26660 Pa, 300 ml de solvant.

Après retour à la pression atmosphérique on introduit sous azote 8,3 g d'hydrure de sodium à 80 % et on maintient à 110 °C sous azote pendant 30 minutes. Puis on réduit la température à 80 °C. On commence alors l'introduction progressive de 41,6 g d'isocyanate de stéaryle pendant 4 heures à 80 °C puis on monte la température de 80 °C à 130 °C en 2 heures et on maintient 2 heures à 130 °C. Après refroidissement à 90 °C, décantation et séchage, on obtient avec un rendement de 100 % une poudre de polyamide 6 présentant les caractéristiques suivantes :
- structure "rose des sables"
- granulométrie comprise entre 3,6 et 7,2 µm
- diamètre moyen des particules de 5,0 µm
- surface spécifique BET : 9,9 $m^2$/g
- volume poreux : 1,21 $cm^3$/g
- absorption d'huile de lin : 170 % en poids

## Revendications

1. Poudre de polyamide dont les particules élémentaires poreuses ont une surface spécifique supérieure à 9 m2/g et un diamètre moyen compris entre 1 et 20 µm, et sont de forme sensiblement sphéroïdale à structure lamellaire dont les lamelles d'épaisseur inférieure à 0,2 µm, liées les unes aux autres constituent des cavités de formes géométriques évoluant entre la forme conique et la forme pyramidale, les sommets de ces formes géométriques étant dirigés vers le centre des particules et le volume poreux interne aux particules élémentaires étant supérieur à 0,3 m3/g dans le domaine des rayons médians des pores compris entre 0,02 et 0,4 micron, susceptible d'être obtenue par polymérisation anionique en milieu solvant de lactame en présence de catalyseur, d'activateur et d'au moins une amide, dont une est une N, N'-alkylène bisamide, ladite polymérisation étant initiée avec une quantité de lactame et d'amide telle que le solvant est en état de sursaturation à la température d'initiation.

2. Poudre de polyamide selon la revendication 1 caractérisée en ce qu'elle absorbe au minimum 90 % de son poids d'huile de lin.

3. Poudre de polyamide selon la revendication 1 caractérisé en ce que la dispersion granulométrique est comprise entre 1,2 et 2,5.

4. Poudre de polyamide selon l'une des revendications 1 à 3 caractérisée en ce que sa surface spécifique est comprise entre plus de 9 $m^2$/g et 30 $m^2$/g.

5. Poudre selon l'une des revendications 1 à 4, caractérisée en ce que sa densité apparente non tassée est comprise entre 0,12 et 0,22.

6. Poudre selon l'une des revendications 1 à 5 caractérisée en ce que sa densité apparente tassée est comprise entre 0,22 et 0,30.

7. Procédé de fabrication des poudres selon l'une des revendications 1 à 6 consistant en une polymérisation anionique en milieu solvant de lactame en présence de catalyseur, d'activateur et d'au moins une amide, dont une est une N,N'-alkylène bisamide, caractérisé en ce que la polymérisation est initiée avec une quantité de lactame et d'amide telle que le solvant est en état de sursaturation à la température d'initiation.

8. Procédé selon la revendication 7 caractérisé en ce que le solvant est une isoparaffine.

9. Procédé selon l'une des revendications 7 ou 8 dans lequel, avant d'initier la polymérisation, on ajoute au milieu réactionnel une amide primaire.

10. Procédé selon la revendication 9 caractérisé en ce que l'amide primaire contient de 12 à 22 atomes de carbone.

11. Procédé selon l'une des revendications 7 à 10 dans lequel la quantité de N,N'-alkylène bisamide mise en oeuvre est comprise entre 0,001 et 4 moles pour 100 moles de lactame.

12. Procédé selon l'une des revendications 7 à 11 caractérisé en ce que la quantité d'amide primaire mise en oeuvre est inférieure à 0,5 mole pour 100 moles de lactame.

13. Procédé selon l'une des revendications 7 à 12 caractérisé en ce que la température d'initiation de la polymérisation est comprise entre 80 et 130 °C.

## Claims

1. Polyamide powder, the porous elementary particles of which have a specific surface greater than 9 $m^2$/g and a mean diameter of between 1 and 20 µm and are substantially spheroidal in shape with a lamellar structure, the lamellae of which, with a thickness of less than 0.2 µm, bonded to one another, comprise cavities with geometrical shapes varying between the conical shape and the pyramidal shape, the tips of these geometrical shapes being directed towards the centre of the particles and the pore volume internal to the elementary particles being greater than 0.3 $m^3$/g in the range of the median radii of the pores of between 0.02 and 0.4 micron, which is capable of being obtained by anionic polymerization, in a solvent medium, of lactam in the presence of catalyst, activator and at least one amide, one of which is an N,N'-alkylenebisamide, the said polymerization being initiated with an amount of lactam and amide such that the solvent is in the supersaturated state at the initiation temperature.

**2.** Polyamide powder according to Claim 1, characterized in that it absorbs at least 90 % of its weight of linseed oil.

**3.** Polyamide powder according to Claim 1, characterized in that the particle size dispersion is between 1.2 and 2.5.

**4.** Polyamide powder according to one of Claims 1 to 3, characterized in that its specific surface is between more than 9 $m^2/g$ and 30 $m^2/g$.

**5.** Powder according to one of Claims 1 to 4, characterized in that its loose bulk density is between 0.12 and 0.22.

**6.** Powder according to one of Claims 1 to 5, characterized in that its packed bulk density is between 0.22 and 0.30.

**7.** Process for the manufacture of the powders according to one of Claims 1 to 6 which consist of an anionic polymerization, in a solvent medium, of lactam in the presence of catalyst, activator and at least one amide, one of which is an N,N'-alkylenebisamide, characterized in that the polymerization is initiated with an amount of lactam and amide such that the solvent is in the supersaturated state at the initiation temperature.

**8.** Process according to Claim 7, characterized in that the solvent is an isoparaffin.

**9.** Process according to either of Claims 7 and 8 in which, before initiating the polymerization, a primary amide is added to the reaction mixture.

**10.** Process according to Claim 9, characterized in that the primary amide contains from 12 to 22 carbon atoms.

**11.** Process according to one of Claims 7 to 10 in which the amount of N,N'-alkylenebisamide used is between 0.001 and 4 mol per 100 mol of lactam.

**12.** Process according to one of Claims 7 to 11, characterized in that the amount of primary amide used is less than 0.5 mol per 100 mol of lactam.

**13.** Process according to one of Claims 7 to 12, characterized in that the initiation temperature for the polymerization is between 80 and 130 °C.

**Patentansprüche**

**1.** Polyamidpulver dessen einzelne poröse Teilchen eine spezifische Oberfläche von über 9 $m^2/g$ und einen mittleren Durchmesser zwischen 1 und 20 $\mu m$ und eine etwa kugelförmige Form mit lamellenartiger Struktur besitzen, deren miteinander verbundene Lamellen mit einer Dicke von weniger als 0,2 $\mu m$ Aussparungen darstellen, die eine konische bis pyramidale geometrische Form aufweisen, wobei deren Scheitelpunkt dem Mittelpunkt der Teilchen zugewandt ist, und das innere Porenvolumen der einzelnen Teilchen bei einem mittleren Radius der Poren zwischen 0,02 und 0,4 $\mu m$ größer als 0,3 $m^3/g$ ist, und das Polyamidpulver erhältlich ist durch anionische Polymerisation in einem Lactam-Lösungsmittel in Gegenwart eines Katalysators, eines Aktivators und wenigstens eines Amids, wobei eines ein N,N'-Alkylendiamid ist, und die Polymerisation mit einer Lactam- und Amidmenge in Gang gesetzt wird, bei der sich das Lösungsmittel bei der Anfangstemperatur in übersättigtem Zustand befindet.

**2.** Polyamidpulver nach Anspruch 1, dadurch gekennzeichnet, daß es wenigstens 90 % seines Eigengewichts an Leinöl absorbiert.

**3.** Polyamidpulver nach Anspruch 1, dadurch gekennzeichnet, daß die statistische Streuung der Korngröße zwischen 1,2 und 2,5 liegt.

**4.** Polyamidpulver nach einem der Ansprüche 1 bis 3, dadurch gekennzeichnet, daß es eine spezifische Oberfläche von mehr als 9 $m^2/g$ bis 30 $m^2/g$ besitzt.

**5.** Polyamidpulver nach einem der Ansprüche 1 bis 4, dadurch gekennzeichnet, daß es ein nicht verdichtetes Schüttvolumen von 0,12 bis 0,22 besitzt.

**6.** Polyamidpulver nach einem der Ansprüche 1 bis 5, dadurch gekennzeichnet, daß es ein verdichtetes Schüttvolumen von 0,22 bis 0,30 besitzt.

**7.** Verfahren zur Herstellung von Polyamidpulver nach einem der Ansprüche 1 bis 6, bestehend aus einer anionischen Polymerisation in einem Lactam-Lösungsmittel in Gegenwart eines Katalysators, eines Aktivators und wenigstens eines Amids, wobei eines ein N,N'-Alkylendiamid ist, dadurch gekennzeichnet, daß die Polymerisation mit einer Lactam- und Amidmenge in Gang gesetzt wird, bei der sich das Lösungsmittel bei der Anfangstemperatur in übersättigtem Zustand befindet.

**8.** Verfahren nach Anspruch 7, dadurch gekennzeichnet, daß das Lösungsmittel ein Isoparaffin ist.

**9.** Verfahren nach Anspruch 7 oder 8, wobei man dem Reaktionsgemisch vor dem Polymerisationsstart ein primäres Amid zugibt.

**10.** Verfahren nach Anspruch 9, dadurch gekennzeichnet, daß das primäre Amid 12 bis 22 Kohlenstoffatome enthält.

**11.** Verfahren nach einem der Ansprüche 7 bis 10, wobei die eingesetzte Menge an N,N'-Alkylendiamid, zwischen 0,001 und 4 mol pro 100 mol Lactam beträgt.

**12.** Verfahren nach einem der Ansprüche 7 bis 11, dadurch gekennzeichnet, daß die eingesetzte Menge an primärem Amid unter 0,5 mol pro 100 mol Lactam liegt.

**13.** Verfahren nach einem der Ansprüche 7 bis 12, dadurch gekennzeichnet, daß die Anfangstemperatur der Polymerisation zwischen 80 und 130 °C liegt.

PLANCHE UNIQUE

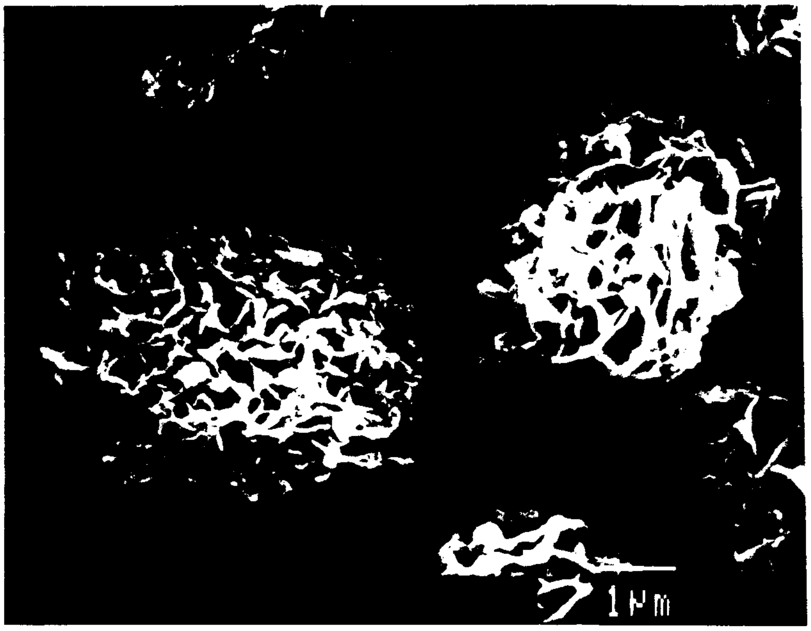


FIGURE 1

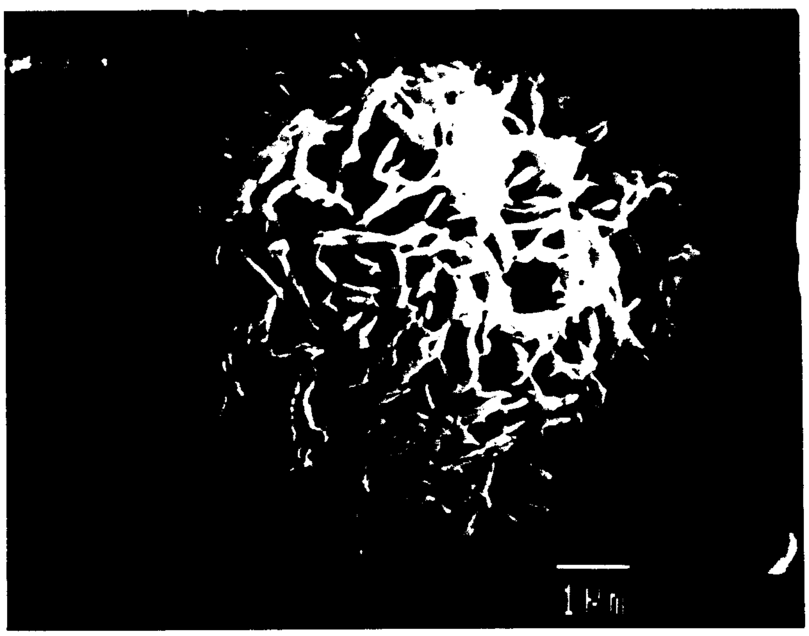


FIGURE 2